# FASCICULE DE BREVET EUROPEEN

(11) **EP 3 193 958 B1**
(45) Date de publication et mention de la délivrance du brevet: **13.05.2020**
(21) Numéro de dépôt: 15759794.9
(22) Date de dépôt: 04.09.2015
(51) Int. Cl.: A61M 1/06

(54) **TIRE-LAIT COMPRENANT DES MOYENS DE RINÇAGE D'UN TUYAU**
BRUSTPUMPE MIT MITTELN ZUM SPÜLEN EINES ROHRES
BREAST PUMP COMPRISING MEANS OF RINSING A TUBE

(30) Priorité: 05.09.2014 FR 1458349
(43) Date de publication de la demande: 26.07.2017
(73) Titulaire: Dorel France, 49309 Cholet Cedex (FR)
(72) Inventeur: RICARD, Adrien, F-49300 Cholet (FR); LECLERE, Jean-Marc, F-49450 Villedieu La Blouere (FR)
(74) Mandataire: Vidon Brevets & Stratégie
(86) Numéro de dépôt international: PCT/EP2015/070304
(87) Numéro de publication internationale: WO 2016/034731

(56) Documents cités:
- EP-A2- 1 468 705
- CN-U- 203 458 634
- US-A- 4 607 596
- US-A1- 2001 044 593

## Description

### 1. Domaine de l'invention

Le domaine de l'invention est celui de la puériculture.

Plus précisément, l'invention concerne les tire-lait, et notamment les tire-lait électriques mettant en œuvre une pompe déportée.

Plus précisément encore, l'invention concerne le rinçage d'un tube, ou tuyau, d'un tel tire-lait dans lequel circule le lait maternel.

### 2. Solutions de l'art antérieur

Il est connu, pour tirer du lait maternel, d'utiliser des dispositifs tire-lait tels que les tire-lait manuels. Ces derniers mettent en œuvre un levier solidaire d'un réservoir collecteur du lait exprimé, le levier étant configuré pour appliquer une dépression entre le réservoir et la poitrine de l'utilisatrice, et ainsi exprimer le lait mammaire.

On connaît également les tire-lait électriques, qui comprennent une unité d'aspiration (ou pompe) reliée à une téterelle destinée à être appliquée sur la poitrine, et une unité de collecte du lait exprimé. La plupart du temps, cette unité de collecte est placée à proximité directe de la téterelle, de façon que le lait s'écoule directement dans celle-ci. Cependant, l'utilisatrice est alors obligée de porter en permanence l'unité de collecte et la pompe associée à proximité de sa poitrine.

Sur certains modèles de tire-lait électriques, il a donc été envisagé de déporter la pompe et/ou l'unité de collecte. Pour ceci, il doit être prévu un tuyau de transfert du lait reliant la téterelle à l'unité de collecte.

De tels appareils étant destinés à collecter du lait maternel pour l'alimentation des nourrissons ou des enfants en bas âge, ils doivent pouvoir être lavés/rincés et stérilisés aisément, notamment afin d'éviter toute transmission de bactéries ou autres germes.

Le nettoyage de tels tire-lait peut par exemple être effectué selon la technique suivante : après démontage de tous les éléments du tire-lait, cette technique connue consiste à rincer et laver les éléments, et notamment le tuyau de transfert du lait, à l'eau propre (ajout de savon possible).

Un inconvénient de cette technique est qu'elle met en œuvre de nombreuses étapes, et qu'elle est donc relativement fastidieuse. En outre, comme indiqué précédemment, il peut être nécessaire de procéder au nettoyage du tire-lait jusqu'à dix fois par jour lors d'une utilisation répétée du dispositif.

Un autre inconvénient de cette technique est la consommation en eau importante qu'elle nécessite. En effet, les étapes de lavage et de rinçage nécessitent une quantité d'eau relativement importante, voire non négligeable lorsque le nettoyage se répète plusieurs fois dans une même journée. Ceci peut engendrer un surcoût pour les utilisateurs.

Encore un autre inconvénient de cette technique est que le lavage du tuyau de transfert du lait n'est pas optimal, du fait que la section du tuyau est faible et que l'eau ne circule pas forcément à l'intérieur du tuyau.

De plus, il n'est pas aisé de contrôler que le nettoyage a été effectué de façon suffisante pour supprimer tous les germes, voire le savon présent dans le tuyau avant le rinçage.

Cependant, les inventeurs ont constaté qu'un simple nettoyage, sans stérilisation, pouvait être suffisant dans certaines circonstances. Ainsi, deux types de nettoyage sont possibles, en fonction de la durée entre deux utilisations du tire-lait.

Plus précisément, il est recommandé de stériliser les éléments, et notamment le tuyau de transfert du lait, lorsque l'intervalle de temps entre deux utilisations est supérieur à 8 heures.

En revanche, pour une durée entre deux utilisations inférieure à 8 heures, un nettoyage (simple rinçage pour le tuyau) après chaque utilisation peut être suffisant (une stérilisation quotidienne est recommandée toutefois).

Il n'existe actuellement aucun dispositif ou technique permettant un lavage/rinçage optimal, simple et efficace, du tuyau de transfert du lait d'un tire-lait.

De même, aucun dispositif ou technique de l'art antérieur ne permet un rinçage automatique de ce tuyau. Le document US2001/0044593 A1 décrit un tire-lait dont les paramètres de la pompe peuvent être ajustés. Le document US4607596 décrit une technique de rinçage d'un tire-lait.

### 3. Objectifs de l'invention

L'invention a notamment pour objectif de pallier au moins certains de ces différents inconvénients de l'art antérieur.

Plus précisément, dans au moins un mode de réalisation, un objectif de l'invention est de fournir un dispositif permettant un rinçage efficace du tuyau de passage du lait d'un tire-lait.

Un autre objectif de l'invention est, selon au moins un mode de réalisation, de fournir un tel dispositif qui soit simple d'utilisation, efficace, robuste, peu coûteux et rapide.

L'invention a aussi pour objectif de fournir, selon au moins un mode de réalisation, un tel dispositif qui ne nécessite qu'une quantité de liquide limitée, tout en assurant un rinçage optimal.

L'invention a également pour objectif de fournir, selon au moins un mode de réalisation, un dispositif permettant un rinçage automatique.

### 4. Exposé de l'invention

Ces objectifs, ainsi que d'autres qui apparaîtront plus clairement par la suite, sont atteints à l'aide d'un tire-lait comprenant une téterelle destinée à être appliquée contre la poitrine d'une mère utilisatrice, un tuyau de passage du lait maternel reliant la téterelle à un réservoir de collecte du lait exprimé, et une pompe destinée à appliquer une dépression dans le tuyau.

Selon l'invention, le tire-lait comprend des moyens de sélection et de commande du mode de fonctionnement de ladite pompe de façon à ce que cette dernière puisse au choix :
- dans au moins un premier mode de fonctionnement, appliquer au moins un premier pompage, faisant varier une dépression, selon des premiers paramètres, dans la téterelle via le tuyau, permettant la collecte du lait lorsque la téterelle est appliquée sur la poitrine de l'utilisatrice de façon à aspirer du lait de ladite téterelle vers ledit réservoir de collecte ;
- dans un deuxième mode de fonctionnement, l'extrémité du tuyau côté téterelle étant plongée dans un liquide de rinçage stocké dans un contenant :
   ∘ appliquer un deuxième pompage, faisant varier la dépression, selon des deuxièmes paramètres distincts des premiers paramètres, dans ledit tuyau de façon à aspirer ledit liquide de rinçage dans ledit tuyau vers ledit réservoir de collecte, pendant un laps de temps fixé entre 10 et 50 secondes et permettant de nettoyer ledit tuyau, puis
   ∘ interrompre le pompage automatiquement.

Ainsi, la pompe du tire-lait est apte à fonctionner selon au moins deux modes de fonctionnement, c'est-à-dire : au moins un premier mode de fonctionnement destiné à exprimer le lait mammaire selon des premiers paramètres, et un deuxième mode de fonctionnement destiné à rincer le tuyau du tire-lait selon des deuxièmes paramètres, distincts des premiers paramètres.

En d'autres termes, les premiers paramètres de pompage permettent d'exprimer, de manière optimale, le lait mammaire tandis que les deuxièmes paramètres, distincts des premiers, garantissent un rinçage parfait du tuyau.

L'invention repose donc sur une approche tout à fait nouvelle et inventive de mise en œuvre, dans un tire-lait, d'une pompe présentant au moins deux modes de fonctionnement sélectionnables. Plus précisément, la pompe présente un mode de fonctionnement spécifique (le deuxième mode de fonctionnement en l'occurrence), dédié uniquement au rinçage du tuyau du tire-lait.

Dans ce deuxième mode de fonctionnement, la pompe comprend un cycle préprogrammé dans lequel les paramètres utilisés (les deuxièmes paramètres) sont choisis pour garantir la décontamination du tuyau du tire-lait lors de son rinçage. Les paramètres du deuxième mode de fonctionnement prévoient en outre un arrêt automatique du cycle, après une durée prédéterminée garantissant le parfait rinçage du tuyau.

Ainsi, le rinçage du tuyau est particulièrement simple, et contrôlé.

Selon un aspect particulier de l'invention, les deuxièmes paramètres du deuxième pompage sont prédéterminés de façon à réaliser un rinçage du tuyau de manière automatique.

Ainsi, la pompe peut présenter un pompage pour le rinçage du tuyau dont les paramètres sont prédéterminés.

Ainsi, la durée du rinçage est suffisante pour garantir que le nettoyage est suffisant et que le tuyau puisse être réutilisé de façon sûre. Il est en effet essentiel que le liquide circule dans le tuyau suffisamment longtemps. Selon l'invention, ceci est garanti par la mise en œuvre d'un pompage pendant le laps de temps fixé, et l'arrêt du pompage est ensuite assuré sans intervention de l'utilisatrice.

Selon un aspect particulier, les premier et deuxième modes de fonctionnement sont programmés automatiquement.

L'opération de rinçage peut nécessiter, selon les modes de réalisation, de désolidariser la téterelle du tuyau et de plonger l'extrémité libre du tuyau dans un récipient contenant un liquide de rinçage. En parallèle, le récipient collecteur de lait est vidé, ou remplacé, pour recevoir le liquide de rinçage.

La pompe est alors amorcée pour aspirer le liquide de rinçage dans le tuyau et le déverser dans le récipient de collecte du liquide sale.

Ainsi, la fonction de rinçage, qui est intégrée au tire-lait (c'est le système de pompage qui assure le rinçage, avec un programme dédié au rinçage), nécessite peu de manipulations, est simple à mettre en œuvre et permet un rinçage optimal du tuyau.

Selon un aspect particulier de l'invention, les moyens de sélection et de commande comprennent au moins deux boutons/touches de sélection, dont un bouton/touche dédié audit deuxième mode de fonctionnement.

Selon un aspect particulier de l'invention, les moyens de sélection et de commande comprennent un commutateur manuel, permettant de sélectionner le ou lesdits premiers modes de fonctionnement ou ledit deuxième mode de fonctionnement.

Selon un aspect particulier de l'invention, dans le deuxième mode de fonctionnement, la valeur de la dépression est comprise entre 12,5 kPa et 15 kPa.

Selon l'invention, dans le deuxième mode de fonctionnement, la durée du cycle de nettoyage est comprise entre 10 et 50 secondes.

Selon un aspect particulier de l'invention, dans le deuxième mode de fonctionnement, la fréquence de la pompe est de 110 cycles/minute.

Selon un aspect particulier de l'invention, la valeur de la dépression, la durée du cycle de nettoyage et la fréquence de la pompe sont fonctions du diamètre interne du tuyau.

Le volume d'eau nécessaire au rinçage du tuyau est également fonction au diamètre interne du tuyau.

Selon un aspect particulier de l'invention, comprend au moins deux voyants lumineux destinés à indiquer le mode de fonctionnement de la pompe.

Selon un aspect particulier de l'invention, les boutons de sélection ou ledit commutateur manuel présentent chacun un élément lumineux destiné à indiquer le mode de fonctionnement de la pompe.

L'invention concerne également un procédé de rinçage d'un tuyau de passage du lait maternel d'un tire-lait comprenant une téterelle destinée à être appliquée contre la poitrine d'une mère utilisatrice, ledit tuyau de passage du lait maternel reliant la téterelle à un réservoir de collecte du lait exprimé, et une pompe destinée à appliquer une dépression dans le tuyau.

Un tel procédé comprend notamment étapes suivantes :
- mise en place de l'extrémité du tuyau côté téterelle dans un liquide de rinçage stocké dans un contenant, la seconde extrémité dudit tuyau restant connectée à ladite pompe ;
- pompage, à l'aide de ladite pompe, dudit liquide de rinçage dans ledit tuyau, dudit contenant vers ledit réservoir de collecte, pendant un laps de temps fixé entre 10 et 50 secondes et permettant de nettoyer ledit tuyau, puis
- interruption automatique dudit pompage.

L'invention propose ainsi une solution particulièrement simple pour le rinçage du tuyau, qu'il n'est pas nécessaire de désolidariser des éléments auxquels il est connecté (selon les modes de réalisation, on peut cependant prévoir une désolidarisation de la téterelle), en exploitant les moyens disponibles (pompe notamment) de façon adaptée.

Ladite étape de pompage peut mettre en œuvre des paramètres de pompage spécifiques à un rinçage du tuyau, présentant au moins une des caractéristiques suivantes :
- valeur de la dépression comprise entre 12,5kPa et 15 kPa ;
- fréquence de la pompe de l'ordre de 110 cycles/minute.

### 5. Liste des figures

D'autres caractéristiques et avantages de l'invention apparaîtront à la lecture de la description suivante d'un mode de réalisation de l'invention, donnée à titre de simple exemple illustratif et non limitatif, et des dessins annexés ci-dessous, parmi lesquels :
- la figure 1 est une représentation schématique du tire-lait selon un mode de réalisation de l'invention ;
- la figure 2 illustre une vue schématique du tire-lait de la figure 1 dans son premier mode de fonctionnement ;
- la figure 3 illustre une vue schématique du tire-lait de la figure 1 dans son deuxième mode de fonctionnement ; et

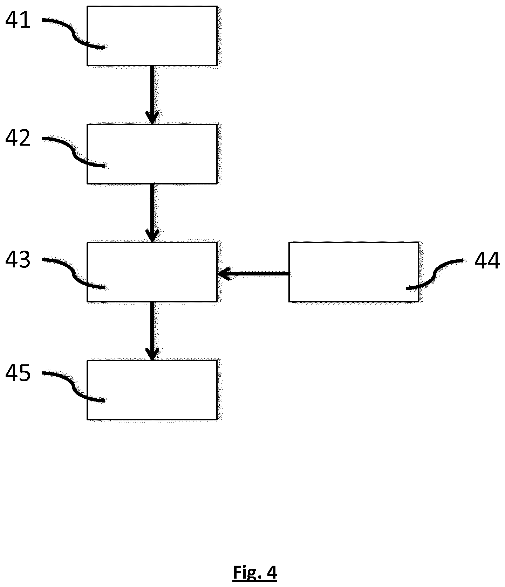

- la figure 4 illustre les différentes étapes du procédé d'utilisation du tire-lait de l'invention.

### 6. Description détaillée d'un mode de réalisation de l'invention

### 6.1. Eléments du tire-lait

Bien qu'il existe des tire-lait présentant des moyens de sélection parmi plusieurs programmes, aucun d'entre eux ne propose de solution simple pour le nettoyage, et en particulier un programme spécifique dédié à ce nettoyage.

L'invention propose donc un tire-lait pour du lait maternel mettant en œuvre une fonction de rinçage à l'eau du tuyau de passage du lait, sans nécessiter le démontage complet du tuyau.

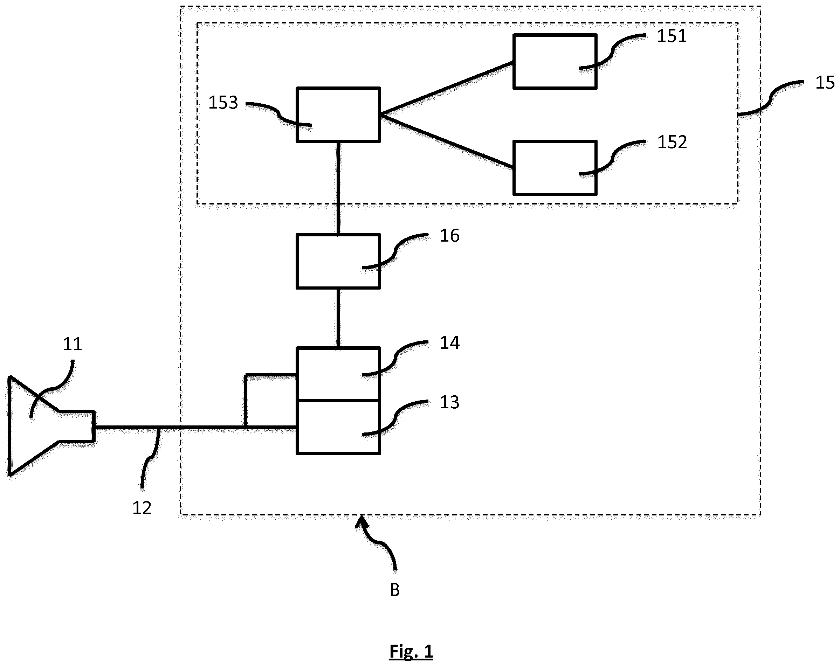

Comme illustré sur le mode de réalisation de la figure 1, le tire-lait 10 de l'invention comprend une téterelle 11, sous la forme d'un entonnoir, destinée à être appliquée contre la poitrine de l'utilisatrice 1. La téterelle 11 est reliée à une première extrémité d'un tuyau 12 souple de passage du lait, le tuyau 12 étant en silicone dans cet exemple. On note que la forme de la téterelle 11 permet son adaptation optimale au sein de l'utilisatrice 1 afin d'assurer une étanchéité suffisante pour l'expression du lait mammaire.

L'autre extrémité du tuyau 12 est raccordée à un réservoir 13 collecteur du lait exprimé. Le réservoir 13 peut être un biberon, par exemple.

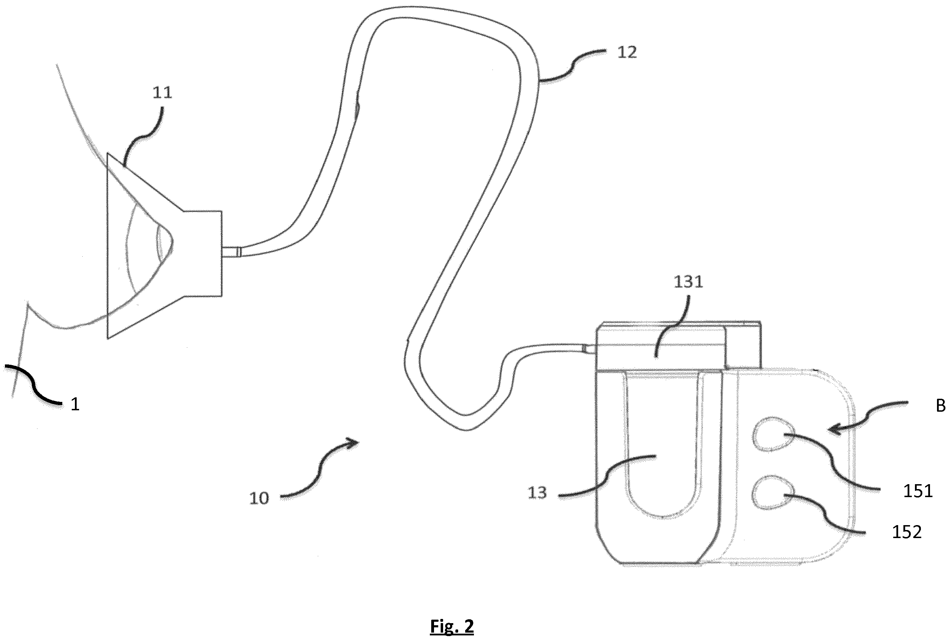

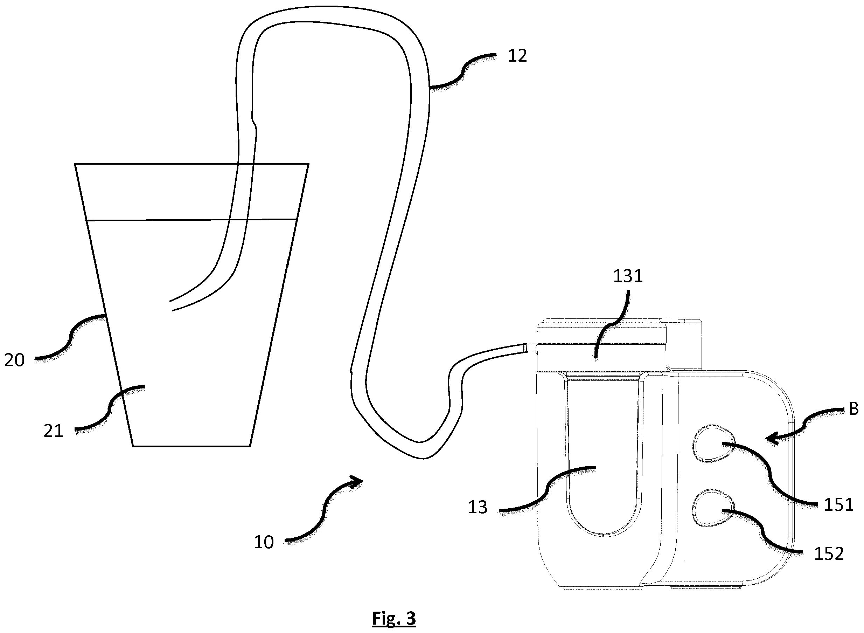

Comme illustré sur les figures 2 et 3, le réservoir 13 est disposé dans un logement correspondant d'un bloc moteur B et est fermé par un couvercle 131 qui porte un élément de raccordement du tuyau 12.

Le bloc moteur B comprend, par ailleurs, une pompe 14 entraînée par un moteur électrique 16 qui permet d'appliquer une dépression dans la téterelle 11 afin d'exprimer le lait mammaire.

Le tuyau 12 permet de transférer le lait depuis la téterelle 11 jusqu'au réservoir 13 collecteur du lait mammaire.

Le tire-lait 10 peut être utilisé selon au moins deux modes, selon que l'utilisatrice souhaite exprimer du lait mammaire (premier(s) mode(s) de fonctionnement, classiques) ou rincer/laver le tuyau 12 de passage du lait (deuxième mode de fonctionnement de l'invention).

Pour ce faire, comme illustré sur les figures 2 et 3, le tire-lait comprend des moyens de sélection et de commande 15 d'un des deux modes de fonctionnement de la pompe 14.

Dans ce mode de réalisation, les moyens de sélection et de commande 15 comprennent deux boutons poussoirs 151 et 152. Ces boutons 151, 152 sont reliés à un contrôleur 153 configuré de manière à faire fonctionner la pompe 14 du tire-lait 10, via le moteur 16, selon deux programmes distincts.

Le contrôleur 153 est configuré pour stocker des paramètres qui définissent un cycle d'opération de la pompe 14 dans chacun des modes de fonctionnement.

Les paramètres comprennent notamment une durée d'aspiration et une valeur de dépression.

Le mode de fonctionnement du tire-lait 10 peut être sélectionné par l'utilisatrice par le biais des boutons 151, 152.

Le bouton 151 d'alimentation du tire-lait 10 permet, lorsqu'il est enfoncé, de lancer le premier mode de fonctionnement classique d'expression du lait. L'activation du bouton 152 permet de déclencher le deuxième mode de fonctionnement, à savoir le rinçage du tuyau.

Le tire-lait 10 peut comprendre deux voyants lumineux correspondant chacun à un des deux modes de fonctionnement.

Dans un mode de réalisation particulier, les boutons 151, 152 sont des boutons lumineux.

### 6.2. Premier mode de fonctionnement : expression du lait

La figure 2 illustre le tire-lait 10 dans son premier mode de fonctionnement classique d'expression du lait.

Dans ce mode de fonctionnement, la téterelle 11 est appliquée contre le sein de l'utilisatrice 1.

La dépression produite par la pompe 14, une fois le bouton 151 activé, permet l'expression du lait maternel qui est ensuite conduit jusqu'au réservoir de collecte 13 par l'intermédiaire du tuyau 12.

L'opération d'expression du lait s'achève automatiquement après un temps prédéterminé. Il peut être envisagé que l'utilisatrice arrête elle-même (manuellement) l'opération d'expression du lait en appuyant sur le bouton 151. De même, une combinaison d'actions possibles manuelle ou automatique peut être envisagée.

Ce premier mode de fonctionnement est classique, les paramètres utilisés pour exprimer le lait mammaire, c'est-à-dire les premiers paramètres, étant connus de l'homme du métier.

Par exemple, les premiers paramètres peuvent comprendre une valeur de dépression comprise entre 25 kPa et 40 kPa, une fréquence de la pompe d'environ 50 cycles/minutes, et une durée du programme d'expression du lait d'environ 30 minutes.

### 6.3. Deuxième mode de fonctionnement : rinçage du tuyau

La figure 3 représente le tire-lait 10 dans son deuxième mode de fonctionnement, destiné à rincer/laver le tuyau 12 souple après son utilisation selon le premier mode de fonctionnement.

Lorsque l'opération d'expression du lait mammaire est terminée, il est important que le tuyau 12 dans lequel passe le lait (et les autres éléments du tire-lait de préférence) soit rincé/lavé. Comme souligné précédemment, ce rinçage du tuyau 12, qui doit avoir lieu entre chaque utilisation, est généralement peu aisé.

Le tire-lait 10 de l'invention permet avantageusement un rinçage automatique du tuyau 12 de passage du lait.

Pour ce faire, il est préalablement nécessaire :
- de changer le réservoir 13 de collecte du lait mammaire exprimé par un réservoir vide ;
- de désolidariser la téterelle 11 du tuyau 12 (dans une variante, on peut imaginer que la téterelle 11 reste en place (il faut seulement que le récipient soit suffisamment grand)) ;
- de plonger l'extrémité laissée libre du tuyau 12 dans un récipient 20 contenant une quantité prédéterminée de liquide de rinçage 21.

Ainsi, cette opération nécessite peu de manipulations.

Le liquide de rinçage 21 utilisé pour le rinçage du tuyau 12 peut être de l'eau ou tout autre produit de rinçage adapté. De préférence l'eau doit être froide.

Une fois ces opérations effectuées, l'utilisatrice peut lancer le rinçage du tuyau 12 en appuyant sur le bouton 152. Plus précisément, le lancement du rinçage correspond au lancement d'un cycle préprogrammé et automatique (y compris l'arrêt) de la pompe 14 qui garantit la décontamination du tuyau 12 du tire-lait.

Le lancement de ce cycle déclenche, par le biais du contrôleur 153, la mise en route de la pompe 14 pendant une durée prédéterminée, égale à environ 30 secondes dans cet exemple qui correspond à un tuyau 12 de diamètre interne d'environ 3 mm.

Le programme de rinçage comprend un arrêt automatique du rinçage. Ce n'est donc pas à l'utilisatrice d'arrêter manuellement le programme. Cet arrêt automatique est le garant du parfait rinçage du tuyau.

Toutefois, et si nécessaire, l'utilisatrice peut appuyer une nouvelle fois sur le bouton 152 pour amorcer un nouveau cycle de rinçage.

Pendant le rinçage, la pompe 14 produit une dépression dans le tuyau 12 permettant ainsi au liquide de rinçage 21 présent dans le récipient 20 de circuler dans le tuyau 12, puis d'être déversé dans le réservoir 13.

Ce deuxième mode de fonctionnement de la pompe, spécifique au rinçage du tuyau du tire-lait, peut présenter des paramètres différents/distincts des premiers paramètres utilisés classiquement pour l'expression du lait. Ces paramètres particuliers, appelé deuxièmes paramètres, ont été définis grâce à des études scientifiques attestant des résultats de ce rinçage. Ces deuxièmes paramètres, non modifiables par l'utilisateur, sont utilisés dans la programmation du deuxième mode de fonctionnement, il ne s'agit donc pas d'une programmation du rinçage par l'utilisatrice.

A titre d'exemple, les deuxièmes paramètres comprennent une valeur de dépression comprise entre 12,5 kPa et 15 kPa et une fréquence de la pompe de l'ordre de 110 cycles par minutes (+/- 10%). La durée d'un cycle de rinçage/nettoyage est comprise entre 10 et 50 secondes (de préférence 30 secondes).

Le volume de liquide de rinçage passant dans le tuyau 12 au cours de ce cycle de rinçage est égal, dans cet exemple, à 120 ml.

Il est à noter que les valeurs de dépression, de fréquence, de durée de rinçage et de volume de liquide de rinçage sont fonctions du diamètre interne du tuyau 12.

L'opération s'achève automatiquement, lorsque le laps de temps nécessaire au rinçage (correspondant à la durée du cycle de nettoyage) est écoulé.

Ceci garantit la propreté du tuyau 12 et permet sa réutilisation sans risque de contamination dans les sept heures qui suivent son rinçage.

### 6.4. Exemple d'utilisation du tire-lait

Comme décrit précédemment, le tire-lait 10 de l'invention comprend des moyens de sélection et de commande 15 du mode de fonctionnement de la pompe 14. Ces moyens de sélection 15, comprenant notamment deux boutons poussoirs 151 et 152, permettent à l'utilisatrice de choisir entre des premiers modes de fonctionnement de la pompe dédiés à l'expression du lait mammaire, et un deuxième mode de fonctionnement spécifique au rinçage du tire-lait 10.

Les premiers modes de fonctionnement de la pompe 14 utilisent des premiers paramètres de fonctionnement permettant d'exprimer le lait mammaire de manière optimale et en prenant compte des aspects physiologiques de l'utilisatrice.

Ainsi, la sélection d'un des premiers modes de fonctionnement est effectuée, lorsque la téterelle 11 est appliquée sur la poitrine de l'utilisatrice, par une pression de l'utilisatrice sur le bouton poussoir 151.

Cette pression du bouton poussoir 151 déclenche un premier pompage, faisant varier une dépression, selon les premiers paramètres, dans la téterelle 11 de façon à aspirer du lait de la téterelle 11 vers le réservoir de collecte 13.

Lorsque le cycle d'expression du lait est terminé, le tuyau doit être rincé (voire, si nécessaire, stérilisé). Le deuxième mode de fonctionnement spécifique au rinçage peut alors être mis en œuvre.

Pour ce faire, on effectue, selon un mode de réalisation, les opérations suivantes :
- changer (41) le réservoir de collecte 13 comprenant du lait exprimé par un réservoir de collecte 13 vide (ou vider le réservoir 13) ; et
- plonger (42), soit la téterelle 11, soit l'extrémité correspondante du tuyau 12 si la téterelle 11 a été désolidarisée, dans un liquide de rinçage 21 stocké dans un contenant 20 (qui peut le cas échéant être un récipient quelconque) ;
- effectuer un pompage (43), dit deuxième pompage, du liquide de rinçage, pendant un laps de temps prédéterminé.

Il suffit pour ceci de sélectionner (44) le deuxième mode de fonctionnement de la pompe, par exemple en pressant le bouton poussoir 152. Cette action déclenche alors le deuxième pompage, faisant varier la pression, selon des deuxièmes paramètres distincts des premiers paramètres, de façon à aspirer le liquide de rinçage 21 dans le tuyau 12 vers le réservoir de collecte 13.

Le programme/cycle de rinçage s'arrête/est interrompu (45) automatiquement après un laps de temps prédéterminé, fonction des deuxièmes paramètres.

L'invention propose ainsi une solution particulièrement simple pour le rinçage du tuyau, qu'il n'est pas nécessaire de désolidariser des éléments auxquels il est connecté (selon les modes de réalisation, on peut cependant prévoir une désolidarisation de la téterelle), en exploitant les moyens disponibles (pompe notamment) de façon adaptée.

### 6.5. Autres aspects et variantes

Dans une variante, les moyens de sélection et de commande 15 de la pompe 14 peuvent comprendre un commutateur manuel (bouton tournant ou levier à deux positions), par exemple.

L'eau utilisée pour le rinçage du tuyau est de préférence à température ambiante ou froide (l'eau tiède ou chaude est moins efficace et peut ne pas garantir l'élimination complète de germes et bactéries).

## Revendications

1. Tire-lait (10) comprenant une téterelle (11) destinée à être appliquée contre la poitrine d'une mère utilisatrice, un tuyau (12) de passage du lait maternel reliant la téterelle (11) à un réservoir de collecte (13) du lait exprimé, et une pompe (14) destinée à appliquer une dépression dans le tuyau (12),
**caractérisé en ce qu'**il comprend des moyens de sélection et de commande (15) du mode de fonctionnement de ladite pompe (14) de façon à ce que cette dernière puisse au choix :
- dans au moins un premier mode de fonctionnement, appliquer au moins un premier pompage, faisant varier une dépression, selon des premiers paramètres, dans la téterelle (11) via le tuyau (12), permettant la collecte du lait lorsque la téterelle (11) est appliquée sur la poitrine de l'utilisatrice de façon à aspirer du lait de ladite téterelle (11) vers ledit réservoir de collecte (13) ;
- dans un deuxième mode de fonctionnement, l'extrémité du tuyau (12) côté téterelle (11) étant plongée dans un liquide de rinçage (21) stocké dans un contenant(20) :
∘ appliquer un deuxième pompage, faisant varier la dépression, selon des deuxièmes paramètres distincts des premiers paramètres, dans ledit tuyau (12) de façon à aspirer ledit liquide de rinçage (21) dans ledit tuyau (12) vers ledit réservoir de collecte (13), pendant un laps de temps fixé entre 10 et 50 secondes et permettant de nettoyer ledit tuyau (12), puis
∘ interrompre le pompage automatiquement.

2. Tire-lait (10) selon la revendication 1, **caractérisé en ce que** les deuxièmes paramètres du deuxième pompage sont prédéterminés de façon à réaliser un rinçage du tuyau (12) de manière automatique.

3. Tire-lait (10) selon la revendication 1 ou 2, **caractérisé en ce que** les moyens de sélection et de commande (15) comprennent au moins deux boutons/touches de sélection (151, 152), dont un bouton/touche (152) dédié audit deuxième mode de fonctionnement.

4. Tire-lait (10) selon la revendication 1 ou 2, **caractérisé en ce que** les moyens de sélection et de commande (15) comprennent un commutateur manuel, permettant de sélectionner le ou lesdits premiers modes de fonctionnement ou ledit deuxième mode de fonctionnement.

5. Tire-lait (10) selon l'une des revendications 1 à 4, **caractérisé en ce que**, dans le deuxième mode de fonctionnement, la valeur de la dépression est comprise entre 12,5kPa et 15 kPa.

6. Tire-lait (10) selon l'une des revendications 1 à 5, **caractérisé en ce que**, dans le deuxième mode de fonctionnement, la fréquence de la pompe (14) est de 110 cycles/minute.

7. Tire-lait (10) selon l'une des revendications 1 à 6, **caractérisé en ce que** la valeur de la dépression, la durée du cycle de nettoyage et la fréquence de la pompe (14) sont fonctions du diamètre interne du tuyau (12).

8. Tire-lait (10) selon l'une des revendications 1 à 7, **caractérisé en ce qu'**il comprend au moins deux voyants lumineux destinés à indiquer le mode de fonctionnement de la pompe.

9. Tire-lait (10) selon l'une des revendications 3 à 7, **caractérisé en ce que** les boutons de sélection (151, 152) ou ledit commutateur manuel présentent chacun un élément lumineux destiné à indiquer le mode de fonctionnement de la pompe.

10. Procédé de rinçage d'un tuyau (12) de passage du lait maternel d'un tire-lait comprenant une téterelle (11) destinée à être appliquée contre la poitrine d'une mère utilisatrice, ledit tuyau (12) de passage du lait maternel reliant la téterelle (11) à un réservoir de collecte (13) du lait exprimé, et une pompe (14) destinée à appliquer une dépression dans le tuyau (12),
**caractérisé en ce qu'**il comprend les étapes suivantes :
- mise en place (42) de l'extrémité du tuyau (12) côté téterelle (11) dans un liquide de rinçage (21) stocké dans un contenant (20), la seconde extrémité dudit tuyau restant connectée à ladite pompe (14) ;
- pompage (43), à l'aide de ladite pompe, dudit liquide de rinçage (21) dans ledit tuyau (12), dudit contenant (21) vers ledit réservoir de collecte (13), pendant un laps de temps fixé entre 10 et 50 secondes et permettant de nettoyer ledit tuyau (12), puis
- interruption (44) automatique dudit pompage.

11. Procédé de rinçage selon la revendication 10, **caractérisé en ce que** ladite étape de pompage met en œuvre des paramètres de pompage spécifiques à un rinçage du tuyau, présentant au moins une des caractéristiques suivantes :
- valeur de la dépression comprise entre 12,5kPa et 15 kPa ;
- fréquence de la pompe de l'ordre de 110 cycles/minute.

## Patentansprüche

1. Brustpumpe (10) umfassend eine Brusthaube (11), die zur Auflage auf die Brust einer anwendenden Mutter bestimmt ist, einen Schlauch (12) zum Durchleiten von Muttermilch, der die Brusthaube (11) mit einem Behälter zum Sammeln (13) der abgepumpten Milch verbindet, und eine Pumpe (14), die dazu bestimmt ist, einen Unterdruck im Schlauch (12) anzulegen,
**dadurch gekennzeichnet, dass** sie Mittel zur Auswahl und zur Steuerung (15) der Funktionsweise der Pumpe (14) umfasst, damit diese Letztere wahlweise:
- in mindestens einer ersten Funktionsweise mindestens einen ersten Pumpvorgang ausführen kann, indem gemäß ersten Parametern ein Unterdruck in der Brusthaube (11) über den Schlauch (12) variiert wird, wodurch das Sammeln der Milch ermöglicht wird, wenn die Brusthaube (11) auf die Brust der Anwenderin aufgelegt wird, um Milch von der Brusthaube (11) in den Sammelbehälter (13) zu saugen;
- in einer zweiten Funktionsweise, in welcher das an der Brusthaube (11) befindliche Ende des Schlauchs (12) in eine Spülflüssigkeit (21), die in einem Behälter (20) aufbewahrt wird, getaucht ist:
∘ einen zweiten Pumpvorgang ausführen kann, indem gemäß zweiten Parametern, die sich von den ersten Parametern unterscheiden, der Unterdruck in dem Schlauch (12) variiert wird, um die Spülflüssigkeit (21) in dem Schlauch (12) in den Sammelbehälter (13) zu saugen, während einer zwischen 10 und 50 Sekunden festgelegten Zeitspanne, und um die Reinigung des Schlauchs (12) zu ermöglichen, danach
∘ den Pumpvorgang automatisch beenden kann.

2. Brustpumpe (10) nach Anspruch 1, **dadurch gekennzeichnet, dass** die zweiten Parameter des zweiten Pumpvorgangs so voreingestellt sind, um ein Spülen des Schlauchs (12) automatisch auszuführen.

3. Brustpumpe (10) nach Anspruch 1 oder 2, **dadurch gekennzeichnet, dass** die Mittel zur Auswahl und zur Steuerung (15) mindestens zwei Knöpfe/Tasten zur Auswahl (151, 152) umfassen, von denen ein/e Knopf/Taste (152) für die zweite Funktionsweise vorgesehen ist.

4. Brustpumpe (10) nach Anspruch 1 oder 2, **dadurch gekennzeichnet, dass** die Mittel zur Auswahl und zur Steuerung (15) einen Handschalter umfassen, mit dem die Auswahl der ersten Funktionsweise oder Funktionsweisen oder der zweiten Funktionsweise möglich ist.

5. Brustpumpe (10) nach einem der Ansprüche 1 bis 4, **dadurch gekennzeichnet, dass** in einer zweiten Funktionsweise der Wert des Unterdrucks zwischen 12,5 kPa und 15 kPa liegt.

6. Brustpumpe (10) nach einem der Ansprüche 1 bis 5, **dadurch gekennzeichnet, dass** in einer zweiten Funktionsweise die Frequenz der Pumpe (14) 110 Zyklen/Minute beträgt.

7. Brustpumpe (10) nach einem der Ansprüche 1 bis 6, **dadurch gekennzeichnet, dass** der Wert des Unterdrucks, die Dauer des Reinigungszyklus und die Frequenz der Pumpe (14) vom Innendurchmesser des Schlauchs (12) abhängig sind.

8. Brustpumpe (10) nach einem der Ansprüche 1 bis 7, **dadurch gekennzeichnet, dass** sie mindestens zwei Leuchtanzeigen umfasst, die dazu bestimmt sind, die Funktionsweise der Pumpe anzuzeigen.

9. Brustpumpe (10) nach einem der Ansprüche 3 bis 7, **dadurch gekennzeichnet, dass** die Knöpfe zur Auswahl (151, 152) oder der Handschalter jeder ein Leuchtelement aufweisen, das dazu bestimmt ist, die Funktionsweise der Pumpe anzuzeigen.

10. Verfahren zum Spülen eines Schlauchs (12) zum Durchleiten von Muttermilch einer Brustpumpe, die eine Brusthaube (11) umfasst, die zur Auflage auf die Brust einer anwendenden Mutter bestimmt ist, wobei der Schlauch (12) zum Durchleiten von Muttermilch die Brusthaube (11) mit einem Behälter zum Sammeln (13) der abgesaugten Milch verbindet, und eine Pumpe (14), die dazu bestimmt ist, einen Unterdruck in dem Schlauch (12) anzulegen,
**dadurch gekennzeichnet, dass** es die folgenden Schritte umfasst:
- Platzierung (42) des Endes des Schlauchs (12), das sich an der Brusthaube (11) befindet, in einer Spülflüssigkeit (21), die in einem Behälter (20) aufbewahrt wird, wobei das zweite Ende des Schlauchs mit der Pumpe (14) verbunden bleibt;
- Pumpen (43), mit Hilfe der Pumpe, der Spülflüssigkeit (21) in dem Schlauch (12) aus dem Behälter (21) in den Sammelbehälter (13), während einer zwischen 10 und 50 Sekunden festgelegten Zeitspanne, und um die Reinigung des Schlauchs (12) zu ermöglichen, danach
- automatische Unterbrechung (44) des Pumpvorgangs.

11. Verfahren zum Spülen nach Anspruch 10, **dadurch gekennzeichnet, dass** der Schritt des Pumpvorgangs Pumpparameter ausführt, die für ein Spülen des Schlauchs spezifisch sind, die mindestens eine der folgenden Eigenschaften aufweisen:
- Wert des Unterdrucks zwischen 12,5 kPa und 15 kPa;
- Frequenz der Pumpe mit der Größenordnung von 110 Zyklen/Minute.

## Claims

1. Breast pump (10) comprising a breast shield (11) intended to be applied against the breast of a user mother, a tube (12) for passage of the mother's milk connecting the breast shield (11) to a reservoir (13) collecting the expressed milk, and a pump (14) intended to apply a negative pressure in the tube (12), **characterised in that** it comprises means (15) for selecting and controlling the operating mode of said pump (14) so that the latter can, as desired:
- in at least a first operating mode, applying at least a first pumping, varying a negative pressure, according to first parameters, in the breast shield (11) via the tube (12), enabling milk to be collected when the breast shield (11) is applied to the breast of the user so as to aspirate milk from said breast shield (11) to said collection reservoir (13);
- in a second operating mode, the end of the tube (12) on the breast shield (11) side being immersed in a rinsing liquid (21) stored in a container (20):
--applying a second pumping, varying the negative pressure, according to second parameters distinct from the first parameters, in said tube (12) so as to aspirate said rinsing liquid (21) in said tube (12) towards said collection reservoir (13), during a period of time set between 10 and 50 seconds and enabling said tube (12) to be cleaned, then
-- automatically interrupting the pumping.

2. Breast pump (10) according to claim 1, **characterised in that** the second parameters of the second pumping are predetermined so as to achieve a rinsing of the tube (12) automatically.

3. Breast pump (10) according to claim 1 or 2, **characterised in that** the selection and control means (15) comprise at least two selection buttons/keys (151, 152), including a button/key (152) dedicated to said second operating mode.

4. Breast pump (10) according to claim 1 or 2, **characterised in that** the selection and control means (15) comprise a manual switch, making it possible to select said first operating mode or modes or said second operating mode.

5. Breast pump (10) according any of claims 1 to 4, **characterised in that**, in the second operating mode, the value of the negative pressure is between 12.5 kPa and 15 kPa.

6. Breast pump (10) according to any of claims 1 to 5, **characterised in that**, in the second operating mode, the frequency of the pump (14) is 110 cycles/minute.

7. Breast pump (10) according to any of claims 1 to 6, **characterised in that** the value of the negative pressure, the duration of the cleaning cycle and the frequency of the pump (14) are dependent on the inside diameter of the tube (12).

8. Breast pump (10) according to any of claims 1 to 7, **characterised in that** it comprises at least two light indictors intended to indicate the operating mode of the pump.

9. Breast pump (10) according to any of claims 3 to 7, **characterised in that** the selection buttons (151, 152) or said manual switch each have a luminous element intended to indicate the operating mode of the pump.

10. Method for rinsing a tube (12) for passage of the breast milk of a breast pump comprising a breast shield (11) intended to be applied against the breast of a user mother, said tube (12) for passage of the mother's milk connecting the breast shield (11) to a collection reservoir (13) for the milk expressed, and a pump (14) intended to apply a negative pressure in the tube (12),
**characterised in that** it comprises the following steps:
- introduction (42) of the end of the tube (12) on the breast shield (11) side into in a rinsing liquid (21) stored in a container (20), said second end of said tube remaining connected to said pump (14):
- pumping (43), thanks to the pump, said rinsing liquid (21) in said tube (12), from said container (21) towards said collection reservoir (13), during a period of time set between 10 and 50 seconds and enabling said tube (12) to be cleaned, then
-- automatically interrupting said pumping.

11. Rinsing method according to claim 10, **characterised in that** said pumping step uses pumping parameters specific to a rinsing of the tube, having at least one of the following characteristics:
- value of the negative pressure between 12.5 kPa and 15 kPa;
- frequency of the pump of around 110 cycles/minute.
